(19) Europäisches Patentamt / European Patent Office / Office européen des brevets

(11) **EP 3 633 379 A1**

(12) **EUROPEAN PATENT APPLICATION**

(43) Date of publication:
**08.04.2020 Bulletin 2020/15**

(51) Int Cl.:
***G01N 33/68*** (2006.01)

(21) Application number: **18198714.0**

(22) Date of filing: **04.10.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Université Nice Sophia Antipolis
  06100 Nice (FR)**
- **Centre National de la Recherche Scientifique
  75016 Paris (FR)**
- **Institut National de la Santé et de la Recherche Médicale
  75013 Paris (FR)**

(72) Inventor: **The designation of the inventor has not yet been filed**

(74) Representative: **Macquet, Christophe
Macquet & Associés
Arche des Dolines
7, rue Soutrane
06560 Sophia Antipolis (FR)**

Remarks:
A request for correction for the claims has been filed pursuant to Rule 139 EPC. A decision on the request will be taken during the proceedings before the Examining Division (Guidelines for Examination in the EPO, A-V, 3.).

(54) **A METHOD FOR DIAGNOSING IN VITRO A BIPOLAR DISORDER OR A MAJOR DEPRESSIVE DISORDER**

(57)     The invention relates to an *in vitro* or ex *vivo* method for diagnosing a bipolar disorder or a major depressive disorder in a patient in a need thereof, comprising the following steps: providing a biological sample from said patient; determining, from said biological sample, the abundance of at least one of the following cytokines IL-17A, TNF-$\alpha$ and IL-10; and diagnosing a bipolar disorder or a major depressive disorder from the determination of the abundance of the at least one of the following cytokine IL-17A, TNF-$\alpha$ and IL-10.

| Biomarker | BD (mean ± SD) | MDD (mean ± SD) | Effect size | p-value | FDR |
|---|---|---|---|---|---|
| CCL2 | 283.7 ± 20.4 | 284.9 ± 14.6 | -0.07 | 0.94 | 0.94 |
| CCL3 | 25.2 ± 7.6 | 16.45 ± 1.1 | 2.02 | 0.90 | 0.94 |
| CCL4 | 105.0 ± 11.9 | 101.2 ± 6.5 | 0.41 | 0.80 | 0.89 |
| CCL11 | 173.3 ± 18.4 | 201.9 ± 18.1 | -1.57 | 0.63 | 0.86 |
| CCL13 | 143.6 ± 14.6 | 139 ± 7.4 | 0.42 | 0.77 | 0.89 |
| CCL17 | 300.5 ± 46.9 | 330.3 ± 26.3 | -0.81 | 0.32 | 0.54 |
| CCL20 | 9.8 ± 1.6 | 8.7 ± 1.1 | 0.87 | 0.15 | 0.39 |
| CCL22 | 1329.0 ± 106.6 | 1249 ± 67.9 | 0.92 | 0.66 | 0.86 |
| CXCL10 | 337.5 ± 32.3 | 333.1 ± 22.2 | 0.16 | 0.82 | 0.89 |
| IL-6 | 2.5 ± 1.4 | 1.11 ± 0.3 | 1.63 | 0.03 | 0.19 |
| IL-7 | 7.4 ± 1.1 | 7.5 ± 1.0 | -0.03 | 0.48 | 0.70 |
| IL-8 | 285.6 ± 147.3 | 109.7 ± 19.0 | 2.12 | 0.04 | 0.21 |
| IL-10 | 0.5 ± 0.1 | 0.31 ± 0.03 | 3.55 | 0.01 | 0.09 |
| IL-12p40 | 91.8 ± 6.4 | 10000 ± 4.8 | 1.71 | 0.15 | 0.39 |
| IL-15 | 1.36 ± 0.04 | 1.29 ± 0.04 | 1.86 | 0.18 | 0.39 |
| IL-16 | 161.1 ± 11.3 | 156.6 ± 9.0 | 0.44 | 0.42 | 0.66 |
| IL-17A | 1.6 ± 0.6 | 0.6 ± 0.1 | 3.09 | 0.0004 | 0.01 |
| IL-27 | 1189.0 ± 110.2 | 979.7 ± 54.2 | 2.55 | 0.08 | 0.28 |
| IFN-$\gamma$ | 5.6 ± 1.1 | 4.4 ± 0.6 | 1.32 | 0.07 | 0.28 |
| TNF-$\alpha$ | 2.1 ± 0.3 | 1.5 ± 0.1 | 3.12 | 0.02 | 0.17 |
| VEGF-A | 144.7 ± 16.9 | 152.6 ± 22.3 | -0.40 | 0.29 | 0.51 |
| sICAM-1 | 548592 ± 27846 | 496136 ± 15423 | 2.42 | 0.11 | 0.34 |
| sVCAM-1 | 654942 ± 27772 | 652033 ± 18229 | 0.13 | 0.75 | 0.89 |
| CRP | 9435000 ± 2440000 | 5698000 ± 1436000 | 1.93 | 0.27 | 0.51 |
| SAA | 22640000 ± 9988000 | 9051000 ± 3032000 | 2.09 | 0.19 | 0.39 |

Fig. 3

**Description**

FIELD OF THE INVENTION

The invention relates to an *in vitro* or ex *vivo* method for diagnosing a bipolar disorder or a major depressive disorder in a patient in a need thereof. It also relates to the use of cytokines as biomarkers of these disorders.

BACKGROUND OF THE INVENTION

Both the major depressive disorder (MDD) and the bipolar disorder (BD) are characterized by mood changes and are therefore referred to as affective disorders. Major depressive disorder is characterized by recurrent episodes of low mood and energy levels. Bipolar disorder is characterized by recurrent and alternating episodes of mood and energy-level disturbances, which are increased on some occasions, for example on mania or hypomania, and decreased on others, for example, on depression. In both major depressive disorder and bipolar disorder, changes in mood are often separated by periods of normal mood, known as euthymia.

The major depressive disorder affects more women than men and its overall lifetime prevalence is 16%. In contrast, the bipolar disorder affects men and women equally, is associated with an earlier age of onset compared to the major depressive disorder, and its prevalence is 4-5-fold lower.

Although mania and hypomania are the most recognizable characteristics of the bipolar disorder, depression is its most frequent clinical presentation. Therefore, the patients suffering from a bipolar disorder are much more likely to present to clinicians when they are depressed, especially in outpatient settings. Unfortunately, the clinical presentation of a patient with bipolar disorder when depressed may not differ from that of a patient suffering from a major depressive disorder. This may explain why almost 40% of bipolar disorder patients are initially misdiagnosed with major depressive disorder and why the average delay for patients suffering from a bipolar disorder to be correctly diagnosed is of approximately 7.5 years.

As a consequence of an initial incorrect diagnosis, bipolar disorder patients are often inappropriately treated with antidepressants alone, which may aggravate the course of the illness and worsen the outcome.

Two self-rated screening instruments, the Mood Disorder Questionnaire (MDQ) and The Hypomania/Mania Symptom Checklist (HCL-32), have been designed to assist clinicians in identifying bipolar disorder patients among those who present with depression. The MDQ has a sensitivity of 0.73 and a specificity of 0.90 indicating that it can correctly identify almost three-quarters of patients with bipolar disorder and will screen out bipolar disorder in 9 of 10 patients without the condition. The HCL-32 has a higher sensitivity of 0.8 but a much lower specificity of 0.51. Despite the usefulness of MDQ and HCL-32 screening instruments, they are unfortunately used by a very limited number of general practitioners in primary care where the majority of care for depression is delivered.

With this objective in mind, several authors have searched for neuroimaging, urine or blood biomarkers that could discriminate major depressive disorder and bipolar disorder patients when they were either in a euthymic, or a depressive state. For example, several authors have used noninvasive structural Magnetic Resonance Imaging (MRI) imaging to identify significant differences in brain morphology between bipolar disorder and major depressive disorder patients. While this approach gave promising results, these results were not adjusted for covariates such as age, gender, and past or current treatments, that depending on the study, identified different regions of the brain as determinant for a differential diagnosis. A result that could possibly be explained by methodological differences or by the inclusion of medicated patients in some studies and of non-medicated patients in others.

Other authors have taken advantage of "-omic" technologies to identify blood or urine biomarkers that were present at different levels in major depressive disorder and bipolar disorder patient body fluids. For example, in a recent exploratory study, a combined gas chromatography-mass spectrometry (GC-MS)-based and nuclear magnetic resonance (NMR) spectroscopic-based metabolomic approach allowed for the identification of six urinary metabolite biomarkers: formate, 2,3-dihydroxybutanoic acid, 2,4-dihydroxypyrimidine, phenylalanine, and $\beta$-alanine; that were present at different levels in the urine of major depressive disorder and bipolar disorder patients. This panel of metabolites discriminated major depressive disorder and bipolar disorder patients with an accuracy of 89.6%. Despite this promising result, and as acknowledged by the authors themselves, there are several notable limitations to this study. First, all subjects were of a particular Chinese ethnicity and were recruited from the same hospital which limits the applicability of the findings. Secondly, the sample size of bipolar disorder subjects was relatively small. Thirdly, results were not adjusted for important confounding variables such as sex, age, body mass index and medication. Then, last but not least, it was not clear whether urine samples were collected from patients when they were depressed.

SUMMARY OF THE INVENTION

Accordingly, a need exists for developing and validating objective laboratory-based tests enabling diagnosis of major depressive disorder or bipolar disorder and, more particularly, differential diagnosis between major depressive disorder and bipolar disorder.

In accordance with a first aspect, the invention concerns an in vitro or ex vivo method for diagnosing a bipolar disorder or a major depressive disorder in a patient in a need thereof, comprising the following steps: providing a biological sample from said patient; determining, from said biological sample, the abundance of at least one of the following cytokines IL-17A, TNF-$\alpha$ and IL-10; and diagnosing a bipolar disorder or a major depressive disorder from the determination of the abundance of the at least one of the following cytokine IL-17A, TNF-$\alpha$ and IL-10.

Preferably, - the patient in a need thereof is a patient showing depressive symptoms - the diagnosing is a differential diagnosing of the bipolar disorder and the major depressive disorder; - the method comprises the steps of: determining, from said biological sample, the abundance of at least two of the following cytokines IL-17A, TNF-$\alpha$ and IL-10; and diagnosing a bipolar disorder or a major depressive disorder from the determination of the abundance of the at least two of the following cytokine IL-17A, TNF-$\alpha$ and IL-10; - the method comprises the steps of: determining, from said biological sample, the abundance of IL-17A, TNF-$\alpha$ and IL-10; and diagnosing a bipolar disorder or a major depressive disorder from the determination of the abundance of IL-17A, TNF-$\alpha$ and IL-10; - the diagnosis is achieved using the determined abundance of one or more additional cytokines selected from the group consisting of IL-6, IL-8, IL-27 and IFN-$\gamma$; - the diagnosis is achieved using at least the determined abundance of IL-17A; - the diagnosis step excludes taking into account the abundance of the following cytokines CCL2, CCL3, CCL4, CCL11, CCL13, CCL17, CCL20, CCL22, CCL26, CXCL10, IL-1-$\alpha$, IL-1-$\beta$, IL-2, IL-4, IL-5, IL-7, IL-8, IL-10, IL-12p40, IL-12p70, IL-13, IL-15, IL-16, IL-21, IL-22, IL-23, IL-31, and TNF-$\beta$ into the provided biological sample; - the biological sample is selected from the group consisting of blood, biopsy tissue, blood serum, blood plasma, stool, sputum, cerebrospinal fluid, or supernatant from cell lysate; - the biological sample is selected from the group consisting of blood, blood plasma or blood serum; and - a probability $p$ that a patient is bipolar is calculated using an equation of the type:

$$p = \frac{1}{1 + e^X}$$

wherein X comprises one or an addition of two or more terms, including at least one of the following terms $\beta_6 F$, $\beta_7 G$, and $\beta_8 H$ wherein the variable "F" is equal to IL-17A serum concentration expressed in pg/ml ; variable "G" is equal to TNF-$\alpha$ serum concentration expressed in pg/ml ; and variable "H" is equal to IL-10 serum concentration expressed in pg/ml; and $\beta_6$ is comprised between 0.281 and 0.343 preferentially equal to approximately 0.312, more preferentially equal to 0.312 ; $\beta_7$ is comprised between 0.112 and 0.136, preferentially equal to approximately 0.124, more preferentially equal to 0.124 ; and $\beta_8$ is comprised between 0.171 and 0.209 preferentially equal to approximately 0.190, more preferentially equal to 0.190.

In accordance with a second aspect, the invention concerns a use of at least one of the following cytokines IL-17A, TNF-$\alpha$ or IL-10 as a biomarker of a bipolar disorder or a major depressive disorder, preferably, the use of at least two of the following cytokines IL-17A, TNF-$\alpha$ or IL-10 as biomarkers of the bipolar disorder or the major depressive disorder and, more preferably, the use of IL-17A, TNF-$\alpha$ and IL-10 as biomarkers of the bipolar disorder or the major depressive disorder.

BRIEF DESCRIPTION OF THE DRAWINGS

Other features and aspects of the present invention will be apparent from the following description and the accompanying drawings, in which:

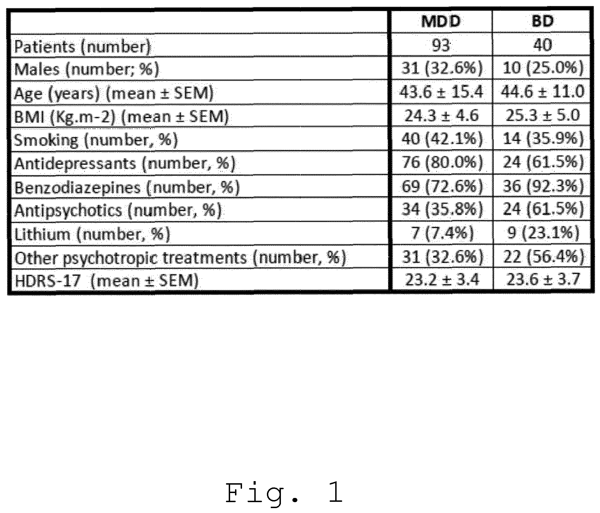

Fig. 1 show a table detailing the patient clinical characteristic that were considered in the Example and, in particular, the total number of patients, numbers of males and females, proportion of males, age, body mass index, tobacco consumption, HDRS-17 score, previous or ongoing treatments with antidepressants, antipsychotics, benzodiazepine and lithium, are shown for all MDE patients as well as for bipolar disorder (BD) and major depressive disorder (MDD) patients;

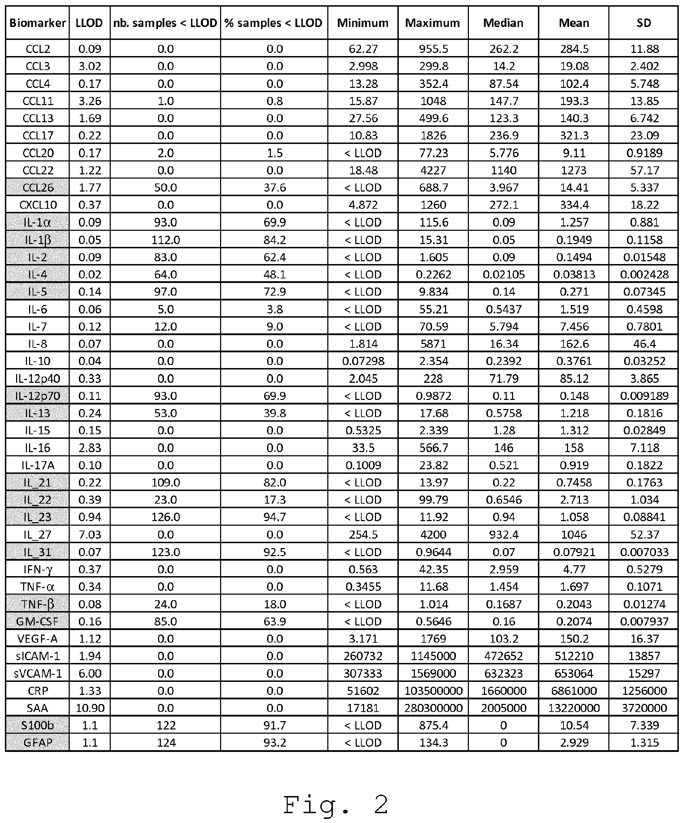

Fig. 2 shows a table with the serum protein concentration descriptive statistics of the patients listed in the table of Fig. 1. In this table, the LLOD (in pg/ml), proportion of samples in which protein levels were < LLOD, the minimum, maximum, median and mean concentrations (in pg/ml), and standard error of the mean (SEM) are indicated;

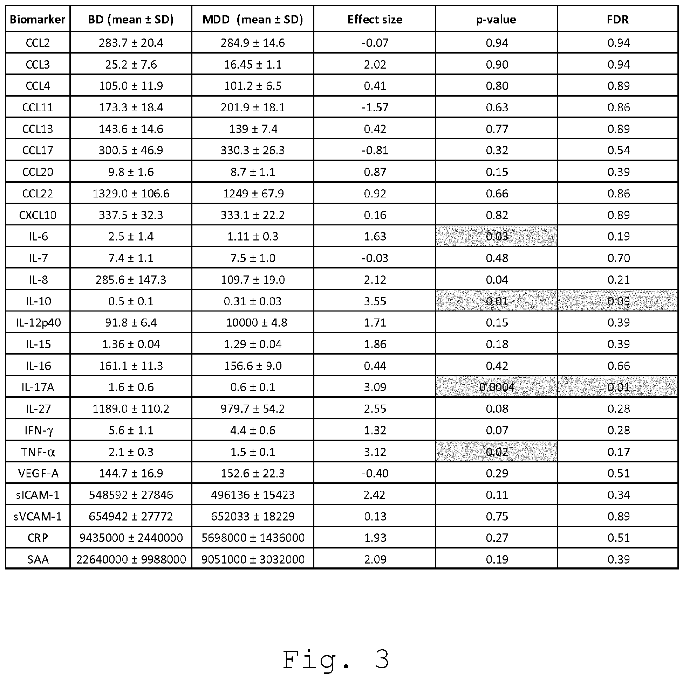

Fig. 3 shows a table comprising the results of the univariate analysis of serum protein levels in BD and MDD patients. For each serum protein, mean concentrations $\pm$ SD in BD and MDD patients are indicated. Effect sizes, p-values and False Discovery Rates (FDRs) are shown; and

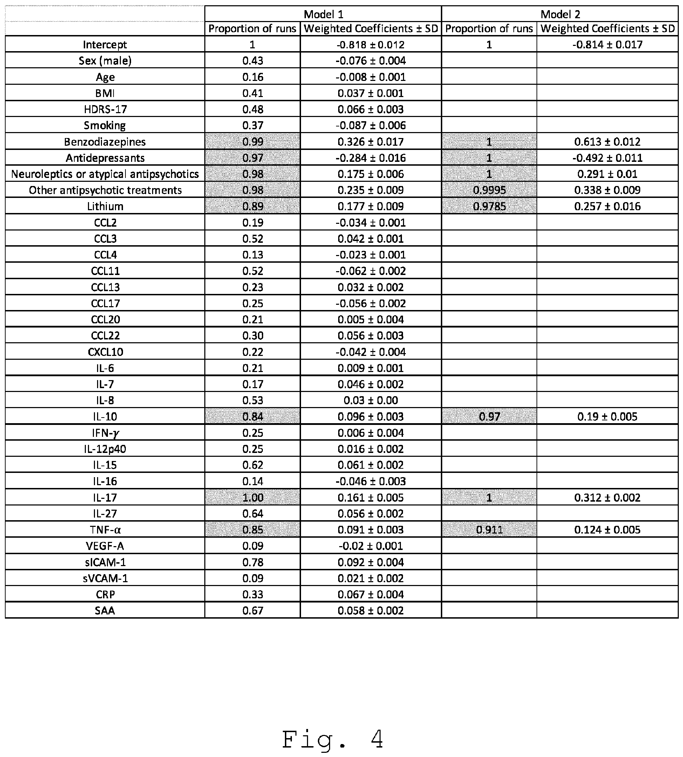

Fig. 4 shows the variables associated with increased odds of belonging to the BD diagnosis category. The data show the list of variables that have been included in the regularized regression logistic model, the mean ($\pm$SD) weighted coefficients. All variables listed in the left column were included in Model 1. Only the variables which have been selected more than 80% of the time in Model 1 were included in Model 2. Alpha and lambda hyper-parameters for Model 1 were 0.167 and 0.268 respectively. Alpha and lambda hyper-parameters for Model 2 were 0.10185 and 0.1023 respectively.

DETAILED DESCRIPTION OF THE INVENTION

The invention concerns a method for diagnosing a bipolar disorder or a major depressive disorder in a patient in a need thereof. More particularly, the invention concerns an *in vitro* or *ex vivo* method for diagnosing a bipolar disorder or a major depressive disorder in a patient in a need thereof, the method comprising a step of determining, from a biological sample of the patient, the abundance of at least one of the following cytokines IL-17A, TNF-$\alpha$ and IL-10."

The major depressive disorder and the bipolar disorder are characterized by mood changes and are therefore referred to as affective disorders. Major depressive disorder is characterized by recurrent episodes of low mood and energy levels. Bipolar disorder is characterized by recurrent and alternating episodes of mood and energy-level disturbances, which are increased on some occasions, for example on mania or hypomania, and decreased on others, for example, on depression. In both major depressive disorder and bipolar disorder, changes in mood are often separated by periods of normal mood, known as euthymia.

According to the invention, the patient is a human which is presenting depressive symptoms. It is to be noted that other diagnosing method may be handled, in particular, upfront the diagnosing method according to the invention in order if a patient is mentally healthy or not.

The invention comprises a first step according to which a biological sample of the patient is provided. The biological sample is preferably selected from the group consisting of blood, biopsy tissue, blood serum, blood plasma, stool, sputum, cerebrospinal fluid, or supernatant from cell lysate. More preferably, it is selected from the group consisting of blood, blood plasma or blood serum. The invention comprises a second step according to which it is determined, from the collected biological sample, the abundance of at least one of the following cytokines biomarkers IL-17A, TNF-$\alpha$ and IL-10. Preferably, it is determined, from said biological sample, the abundance of at least two of the following cytokines IL-17A, TNF-$\alpha$ and IL-10. More preferably, it is determined, from said biological sample, the abundance of IL-17A, TNF-$\alpha$ and IL-10.

IL-17A is a cytokine, that is produced by a subpopulation of CD4+ T cells called Th17 cells. Th17 cells are constitutively present in a part of the gut, the lamina propria of the small intestines, due to a specific population of bacteria present there (segmented filamentous bacteria), where they ensure immune surveillance and proper gut function and are quasi absent in other organs such as lung or liver. Infections or other conditions can increase the Th17 cell population, and once activated Th17 cells promote the eradication of extracellular bacteria, and fungi such as *Candida albicans.* TNF-$\alpha$ is a pro-inflammatory cytokine that is produced by several cell types including T lymphocytes, macrophages, neutrophils, astrocytes, glia cells, fibroblasts, and smooth muscle cells in response to injury and inflammatory stimuli. IL-10 is a cytokine with antiinflammatory properties which has a central role in preventing inflammatory and autoimmune pathologies. While it was originally demonstrated to be produced by CD4+ T helper type 2 (Th2) cells, many immune cell types could produce it, including Th1 and regulatory T cells, CD8+ T cells, B cells, macrophages, dendritic cells, neutrophils and eosinophils. Notably, some nonhematopoietic cell types, such as epithelial cells, can also produce IL-10. IL-10 production in the brain has also been described, but the cellular sources remained to be identified. Several preclinical and clinical studies have suggested a possible role of IL-10 in brain function and behavior.

The abundance of the biomarkers in the blood sample is for example the concentration of said biomarkers in the blood sample, in the serum part of said blood sample or in the plasma part of said blood sample. For measuring the abundance of the plurality of the biomarkers in the collected sample of blood according to the invention, various methods, that are well-known from the man skilled in the art, may be used. For example, these measures may be carried out using electro-chemo-luminescence (ECL)-based or bead-based immunoassays.

The invention comprises a third step according to which it is diagnosed a bipolar disorder or a major depressive disorder from the determination of the abundance of the at least one of the following cytokine IL-17A, TNF-$\alpha$ and IL-10. Preferably, the diagnosing is a differential diagnosing of the bipolar disorder and the major depressive disorder. Preferably, the diagnosing is achieved from the determination of the abundance of at least two of the following cytokine IL-17A, TNF-$\alpha$ and IL-10. More preferably, it is achieved from the determination of the abundance of IL-17A, TNF-$\alpha$ and IL-10. For example, the diagnosis is achieved from the determination of the abundance of one cytokine selected from the group consisting of IL-17A or TNF-$\alpha$ or IL-10; two cytokines selected from the group consisting of IL-17A and/or TNF-$\alpha$ or IL-10; IL-17A, TNF-$\alpha$ and IL-10; or one cytokine selected from the group consisting of IL-17A or TNF-$\alpha$ or IL-10, or two cytokines selected from the group consisting of IL-17A and/or TNF-$\alpha$ or IL-10, or IL-17A, TNF-$\alpha$ and IL-10, and one or more additional cytokines selected from the group consisting of IL-6, IL-8, IL-27 and IFN-$\gamma$, but excluding the following cytokines CCL2, CCL3, CCL4, CCL11, CCL13, CCL17, CCL20, CCL22, CCL26, CXCL10, IL-1-$\alpha$, IL-1- $\beta$, IL-2, IL-4, IL-5, IL-7, IL-8, IL-10, IL-12p40, IL-12p70, IL-13, IL-15, IL-16, IL-21, IL-22, IL-23, IL-31 and TNF- $\beta$.

To that effect, a probability $p$ that a patient is bipolar can be determined. This could be done by replacing the variables "A", "B", "C", "D", "E", "F", "G" and "H" in equation below by the appropriate values as described:

$$p = \frac{1}{1 + e^{\beta_0 + \beta_1 A + \beta_2 B + \beta_3 C + \beta_4 D + \beta_5 E + \beta_6 F + \beta_7 G + \beta_8 H}}$$

wherein:

the variable "A" is equal to 1 if the patient has been treated with benzodiazepine, and equal to 0 in the opposite case ; variable "B" is equal to 1 if the patient has been treated with antidepressants, and equal to 0 in the opposite case ; variable "C" is equal to 1 if the patient has been treated with neuroleptics or atypical antipsychotics, and equal to 0 in the opposite case ; variable "D" is equal to 1 if the patient has been treated with any other antipsychotics, and equal to 0 in the opposite case ; variable "E" is equal to 1 if the patient has been treated with lithium, and equal to 0 in the opposite case ; variable "F" would be equal to IL-17A serum concentration expressed in pg/ml ; variable "G" is equal to TNF-$\alpha$ serum concentration expressed in pg/ml ; and variable "H" is equal to IL-10 serum concentration expressed in pg/ml; and

$\beta_0$ is comprised between -0.895 and -0.733 preferentially equal to approximately -0.814, more preferentially equal to -0.814 ; $\beta_1$ is comprised between 0.552 and 0.674 preferentially equal to approximately 0.613, more preferentially equal to 0.613 ; $\beta_2$ is comprised between -0.541 and -0.443 preferentially equal to approximately -0.492, more preferentially equal to -0.492 ; $\beta_3$ is comprised between 0.262 and 0.320 preferentially equal to approximately 0.291, more preferentially equal to 0.291; $\beta_4$ is comprised between 0.304 and 0.372 preferentially equal to approximately 0.338, more preferentially equal to 0.338 ; $\beta_5$ is comprised between 0.231 and 0.283 preferentially equal to approximately 0.257, more preferentially equal to 0.257 ; $\beta_6$ is comprised between 0.281 and 0.343 preferentially equal to approximately 0.312, more preferentially equal to 0.312 ; $\beta_7$ is comprised between 0.112 and 0.136, preferentially equal to approximately 0.124, more preferentially equal to 0.124 ; and $\beta_8$ is comprised between 0.171 and 0.209 preferentially equal to approximately 0.190, more preferentially equal to 0.190.

The probability $p$ above may be then calculated taking into account that the patient has been treated using benzodiazepine, antidepressants, neuroleptics or atypical antipsychotics, and/or any other antipsychotics, or not. If the calculation of the probability does not take into account treatments of the patient using the above drugs, then the corresponding coefficient $\beta_1$, $\beta_2$, ..., $\beta_5$ are considered as equal to zero. The probability $p$ is finally calculated on the basis of one, two or the three cytokines IL-17A, TNF-$\alpha$ and IL-10, depending of the values of $\beta_6$, $\beta_7$ and $\beta_8$. The additional cytokines IL-6, IL-8, IL-27 and IFN-$\gamma$ may form additional members of the equation, for example to improve the accuracy of the probability $p$ that is calculated. They will be calculated taking into account additional coefficients $\beta_9$, $\beta_{10}$, $\beta_{11}$ and $\beta_{12}$, respectively, and their respective serum concentration I, J, K and L expressed in pg/ml.

Hence, the probability p is calculated using an equation that is of the type:

$$p = \frac{1}{1 + e^X}$$

wherein X comprises one or an addition of two or more terms, including at least one of the following terms $\beta_6 F$, $\beta_7 G$, and $\beta_8 H$.

Thus, according to the invention, at least one, at least two or the three cytokines IL-17A, TNF-$\alpha$ and IL-10 are used as biomarkers of the bipolar disorder, of the major depressive disorder or to achieve a differential diagnosis of bipolar disorders and major depressive disorders. Indeed, those three cytokines have been identified that, when combined, discriminate bipolar and unipolar patients for example after adjustment to past or ongoing treatments with antidepressants, benzodiazepines, antipsychotics or lithium. While these three cytokines have already been associated with affective disorders in humans and behavioral alterations in mice, this study is the first one to demonstrate that they can be used to discriminate BD and MDD patients. The invention pave the way for the development of a blood-based assisted clinical decision support system for the differential diagnosis of bipolar disorder and major depressive disorder patients.

EXAMPLE:

1. Methods and material

Eligible study participants were 148 adults diagnosed with current Major Depressive Episode (MDE) and 100 age- and gender- matched healthy controls from a study registered in ClinicalTrials.gov with ID: NCT02209142. Participants had a clinical evaluation using the Semi-Structured Clinical Interview of the 4th edition of the Diagnostic and Statistical Manual of Mental Disorders (DSM). Patients were recently admitted in a psychiatric unit or have been recently referred to a psychiatrist for a MDE. The diagnosis of MDE was made by skilled psychiatrists based on the DSM-IV criteria of mood disorders and the MDE severity was evaluated by the 17-item Hamilton Depression Rating Scale (HDRS). Patients were included if they scored 19 or higher on the HDRS. Exclusion criteria were a history of substance use disorder in the past 12 months, a diagnosis of schizophrenia, psychotic or schizoaffective disorder according to the DSM-IV, a severe progressive medical disease, pregnancy, vaccination within a month before the inclusion in the study and being under 18. Fourteen patients were excluded due to the manifestation of exclusion criteria during the study (diagnosis of severe medical conditions, consent withdrawal) or unavailable gene expression data and/or main outcome measures. Finally, data from 134 MDE patients were included in the analyses. Former and ongoing patient treatments were recorded including those involving the use of selective serotonin reuptake inhibitors (SSRIs), tricyclic antidepressants (TCAs), monoamine oxidase inhibitors (MAOIs), benzodiazepines, atypical antipsychotics, lithium, sysmotherapy, transcranial magnetic stimulation (TMS), structured psychotherapy, valproate salts, carbamazepine and lamotrigine. All participants received a full explanation of the procedure and signed a written consent form before their participation.

Peripheral blood samples were obtained from fasting subjects between 7:00 am and 9:00 am on workdays. Five milliliters of peripheral blood were drawn by venipuncture into serum Vacutainer tubes. For the serum collection, the blood was allowed to clot for 1 h before centrifugation (1500 x g, 10 min). The serum and plasma samples were stored in 0.5 ml aliquots at -80°C. For the measurements of cytokine and antibody levels, serum samples were thawed on ice, and 50 $\mu$l aliquots were prepared and stored at - 80°C.

Serum levels of CC chemokine ligand (CCL)2, CCL3, CCL4, CCL11, CCL13, CCL17, CCL20, CCL22, CCL26, CXC chemokine ligand (CXCL)10, IL-1-$\alpha$, IL-1-$\beta$, IL-2, IL-4, IL-5, IL-6, IL-7, IL-8, IL-10, IL-12p40, IL-12p70, IL-13, IL-15, IL-16, IL-17A, IL-21, IL-22, IL-23, IL-27, IL-31, interferon (IFN)-y, Tumor Necrosis Factor (TNF)-$\alpha$, TNF-$\beta$, Granulocyte-Macrophage Colony-Stimulating Factor (GM-CSF), Vascular Injury Growth Factor (VEGF)-A, soluble intercellular adhesion molecule (sICAM)-1 and soluble vascular endothelial cell adhesion molecule (sVCAM)-1, C Reactive Protein (CRP), serum amyloide A protein (SAA), S100B and Glial fibrillary acidic protein (GFAP) were measured using the Proinflammatory Panel 1, Cytokine Panel 1, Chemokine Panel 1 Vascular Injury Panel II V-PLEX™ kits (MesoScale Discovery (MSD)). All assays were performed according to the manufacturer's instructions. The data were acquired on the V-PLEXR Sector Imager 2400™ plate reader and analyzed using the Discovery Workbench™ 3.0 software (MSD). The standard curves for each cytokine were generated using the premixed lyophilized standards provided in the kits. Serial 2-fold dilutions of the standards were run to generate a 13-standard concentration set, and the diluent alone was used as a blank. The cytokine concentrations were determined from the standard curve using a 4-paramater logistic curve fit to transform the mean light intensities into concentrations. The Lower Limit Of Detection (LLOD) was a calculated concentration corresponding to the average signal 2.5 standard deviations above the background (zero calibrator).

In univariate analysis, Student's t-test and Mann-Whitney-Wilcoxon test were performed to assess statistical significance of Gaussian and non-Gaussian distributed data respectively. To develop a predictive model for remission we used the elastic net, which is a regularized regression model, i.e. generalized linear model with penalties to avoid extreme parameters that could cause overfitting. Elastic net is also a method of selection of variables that addresses the issue of multicollinearity that arises in the dataset because cytokines and chemokines are not independent of each other. To minimize variation across testing datasets, we repeated 5-fold cross-validation 200 times with independent random dataset partitions to optimize stability. The hyper-parameters alpha and lambda were tuned 10 times for each partition via 5-fold cross-validation with the optimal tuning parameter values chosen to maximize the area under the Receiver Operating Characteristics (ROC) curve (AUC). Weighted mean coefficient values ($\beta$) were calculated using the proportion of drawings in which the coefficient was different from zero (meaning the associated variable was selected as important) as the ponderation. All statistical analyses were performed using the R software packages Stats™, Caret™, Glmnet™, pROC™, eNetXplorer™.

2. Results

2.1 Serum protein levels in MDD and BD patients

As shown in Fig. 1, the MDE patients were extensively characterized for clinical features including age, body-mass-index (BMI), tobacco consumption, past or on-going treatments with antidepressants, antipsychotics, benzodiazepine and lithium. They were also assessed for depression-associated symptoms using the Hamilton Depression Rating Scale (HDRS). The serum samples were analysed for 12 chemokines (CCL2, CCL3, CCL4, CCL11, CCL13, CCL17, CCL20, CCL22, CCL26, CXCL10), 15 interleukins (IL-1-$\alpha$, IL-1-$\beta$, IL-2, IL-4, IL-5, IL-6, IL-7, IL-8, IL-10, IL-12p40, IL-12p70, IL-13, IL-15, IL-16, IL-17A, IL-21, IL-22, IL-23, IL-27, IL-31), three inflammatory cytokines (IFN-$\gamma$, TNF-$\alpha$, TNF-$\beta$, two growth factors (GM-CSF and VEGFA), two proteins produced by liver in response to inflammation (CRP, SAA), two biomarkers of vascular injury (sICAM-1 and sVCAM-1) and two biomarkers of brain-blood barrier (BBB) permeability. As shown in Fig. 2, among the 41 analyzed proteins, 16 were below the LLOD in more than 10% of samples and were not included in downstream analyses. In an exploratory analysis, the levels of the 35 remaining proteins were compared in BD and MDD patients using univariate analysis. After correction for multiple testing, and as shown in Fig. 3, it was found that both IL-17A and IL-10 were expressed at higher levels in BD patients compared to MDD patients. To a lesser extent, it is the case as well for IL-6, IL-8, IL-27, IFN-$\gamma$.

2.2 Multivariate classification

Most cytokines and chemokines belong to common biochemical or functional pathways. Furthermore, their production could be impacted by patient clinical characteristics including age, gender, body mass index (BMI) and tobacco consumption. Most importantly, several studies have suggested that some psychotropic drugs including antidepressants, antipsychotics and lithium may impact serum levels of cytokines and chemokines and other inflammatory biomarkers. In contrast to univariate statistical methods that assess the differential expression of individual proteins without considering the relationships between them or with other variables, classification methods are used to establish a prediction model based on samples with known class outcomes (e.g. BD or MDD). A set of biomarkers with the best joint discriminatory ability to differentiate between the classes is identified (predictor selection), and the resulting prediction model is used to predict the class outcomes of new patient samples. Unlike univariate methods, these multivariable methods consider the relationships between candidate biomarker proteins, and seek to capture the differences between the sample groups on a multi-feature level. We therefore used one of these multivariate methods, termed regularized logistic regression, to identify serum biomarkers that could discriminate BP and MDD patients after adjustment for covariates known or suspected to either impact serum biomarker levels and/or being associated with one of the two diagnosis categories: gender, age, BMI, tobacco consumption, and former or ongoing treatments with antidepressants, antipsychotics, benzodiazepines and lithium and HDRS-17 score. IL-17A was selected as a determinant feature in 1997 runs out of 2000 (Fig. 4). TNF-$\alpha$ and IL-10 were also selected in more that 80% of drawings, i.e. 1849 and 1836 respectively (Fig. 4). High serum levels of these three cytokines were all associated with decreased odds of belonging to the BD diagnosis category and the predictive value of this model was good (mean AUC = 0.698 $\pm$ 0.098; mean sensitivity: 95.51 $\pm$ 2.05%; mean specificity: 18.99 $\pm$ 7.55%).

Ideally, a blood-based diagnosis algorithm should not only be specific and sensitive, but also relying on a small number of biomarkers. As IL-17A, IL-10 and TNF-$\alpha$ were those that were selected the most frequently in random training/test drawings, we further estimated the predictive value of these three combined cytokines after adjustment to medications which were selected as determinant features in Model 1. The predictive value of this new model was higher (mean AUC = 0.80 $\pm$ 0.07), and its sensitivity and specificity were 94.70 $\pm$ 0.01% and 35.17 $\pm$ 0.05% respectively. Altogether, the data show that serum levels of IL-17A and/or IL-10 and/or TNF-$\alpha$, preferably these three cytokines in combination, can serve as diagnosis biomarkers to differentiate MDD and BD patients.

It is to be noted that the Model 2 as appearing in Fig. 4 could be used to determine the probability (p) that a patient is bipolar.

This could be done by replacing the variables "A", "B", "C", "D", "E", "F", "G" and "H" in equation below by the appropriate values as described:

$$p = \frac{1}{1 + e^{-0.814 + 0.613A + -0.492B + 0.291C + 0.338D + 0.257E + 0.312F + 0.124G + 0.190H}}$$

wherein:

the variable "A" is equal to 1 if the patient has been treated with benzodiazepine, and equal to 0 in the opposite case ; variable "B" is equal to 1 if the patient has been treated with antidepressants, and equal to 0 in the opposite case ; variable "C" is equal to 1 if the patient has been treated with neuroleptics or atypical antipsychotics, and equal to 0 in the opposite case ; variable "D" is equal to 1 if the patient has been treated with any other antipsychotics, and equal to 0 in the opposite case ; variable "E" is equal to 1 if the patient has been treated with lithium, and equal to 0 in the opposite case ; variable "F" would be equal to IL-17A serum concentration expressed in pg/ml ; variable "G" is equal to TNF-$\alpha$ serum concentration expressed in pg/ml ; and variable "H" is equal to IL-10 serum concentration expressed in pg/ml.

**Claims**

1. An *in vitro* or ex *vivo* method for a bipolar disorder or a major depressive disorder in a patient in a need thereof, comprising the following steps:

   determining, from a biological sample, the abundance of at least one of the following cytokines IL-17A, TNF-$\alpha$ and IL-10; and
   diagnosing a bipolar disorder or a major depressive disorder from the determination of the abundance of the at least one of the following cytokine IL-17A, TNF-$\alpha$ and IL-10.

2. The method according to claim 1, wherein the patient in a need thereof is presenting depressive symptoms.

3. The method according to any one of the claims 1 or 2, wherein the diagnosing is a differential diagnosing of the bipolar disorder and the major depressive disorder.

4. The method according to any one of the preceding claims, comprising the steps of:

   determining, from said biological sample, the abundance of at least two of the following cytokines IL-17A, TNF-$\alpha$ and IL-10; and
   diagnosing a bipolar disorder or a major depressive disorder from the determination of the abundance of the at least two of the following cytokine IL-17A, TNF-$\alpha$ and IL-10.

5. The method according to any one of the preceding claims, comprising the steps of:

   determining, from said biological sample, the abundance of IL-17A, TNF-$\alpha$ and IL-10; and
   diagnosing a bipolar disorder or a major depressive disorder from the determination of the abundance of IL-17A, TNF-$\alpha$ and IL-10.

6. The method according to any one of the preceding claims, wherein the diagnosis is achieved using the determined abundance of one or more additional cytokines selected from the group consisting of IL-6, IL-8, IL-27 and IFN-$\gamma$.

7. The method according to any one of the preceding claims, wherein the diagnosis is achieved using at least the determined abundance of IL-17A.

8. The method according to any one of the preceding claims, wherein the diagnosing step excludes taking into account the abundance of the following cytokines CCL2, CCL3, CCL4, CCL11, CCL13, CCL17, CCL20, CCL22, CCL26, CXCL10, IL-1-$\alpha$, IL-1-$\beta$, IL-2, IL-4, IL-5, IL-7, IL-8, IL-10, IL-12p40, IL-12p70, IL-13, IL-15, IL-16, IL-21, IL-22, IL-23, IL-31 and TNF-$\beta$ into the provided biological sample.

9. The method according to any one of the preceding claims, wherein the biological sample is selected from the group consisting of blood, biopsy tissue, blood serum, blood plasma, stool, sputum, cerebrospinal fluid, and supernatant from cell lysate.

10. The method according to claim 8, wherein the biological sample is selected from the group consisting of blood, blood plasma and blood serum.

11. The method according to any one of the preceding claims, wherein a probability p that a patient is bipolar is calculated using an equation of the type: $$p = \frac{1}{1 + e^X}$$

    wherein X comprises one or an addition of two or or more terms, including at least one of the following terms $\beta_6$F, $\beta_7$G, and $\beta_8$H wherein the variable "F" is equal to IL-17A serum concentration expressed in pg/ml ; variable "G" is equal to TNF-$\alpha$ serum concentration expressed in pg/ml ;
    and variable "H" is equal to IL-10 serum concentration expressed in pg/ml; and $\beta_6$ is comprised between 0.281 and 0.343 preferentially equal to approximately 0.312, more preferentially equal to 0.312 ; $\beta_7$ is comprised between 0.112 and 0.136, preferentially equal to approximately 0.124, more preferentially equal to 0.124 ; and $\beta_8$ is comprised between 0.171 and 0.209 preferentially equal to approximately 0.190, more preferentially equal to 0.190.

12. A use of at least one of the following cytokines IL-17A, TNF-$\alpha$ or IL-10 as a biomarker of a bipolar disorder or a major depressive disorder.

13. The use of claim 12, of at least two of the following cytokines IL-17A, TNF-$\alpha$ or IL-10 as biomarkers of the bipolar disorder or the major depressive disorder.

14. The use of claim 13, of the IL-17A, TNF-$\alpha$ and IL-10 as biomarkers of the bipolar disorder or the major depressive disorder.

|  | MDD | BD |
|---|---|---|
| Patients (number) | 93 | 40 |
| Males (number; %) | 31 (32.6%) | 10 (25.0%) |
| Age (years) (mean ± SEM) | 43.6 ± 15.4 | 44.6 ± 11.0 |
| BMI (Kg.m-2) (mean ± SEM) | 24.3 ± 4.6 | 25.3 ± 5.0 |
| Smoking (number, %) | 40 (42.1%) | 14 (35.9%) |
| Antidepressants (number, %) | 76 (80.0%) | 24 (61.5%) |
| Benzodiazepines (number, %) | 69 (72.6%) | 36 (92.3%) |
| Antipsychotics (number, %) | 34 (35.8%) | 24 (61.5%) |
| Lithium (number, %) | 7 (7.4%) | 9 (23.1%) |
| Other psychotropic treatments (number, %) | 31 (32.6%) | 22 (56.4%) |
| HDRS-17 (mean ± SEM) | 23.2 ± 3.4 | 23.6 ± 3.7 |

# Fig. 1

| Biomarker | LLOD | nb. samples < LLOD | % samples < LLOD | Minimum | Maximum | Median | Mean | SD |
|---|---|---|---|---|---|---|---|---|
| CCL2 | 0.09 | 0.0 | 0.0 | 62.27 | 955.5 | 262.2 | 284.5 | 11.88 |
| CCL3 | 3.02 | 0.0 | 0.0 | 2.998 | 299.8 | 14.2 | 19.08 | 2.402 |
| CCL4 | 0.17 | 0.0 | 0.0 | 13.28 | 352.4 | 87.54 | 102.4 | 5.748 |
| CCL11 | 3.26 | 1.0 | 0.8 | 15.87 | 1048 | 147.7 | 193.3 | 13.85 |
| CCL13 | 1.69 | 0.0 | 0.0 | 27.56 | 499.6 | 123.3 | 140.3 | 6.742 |
| CCL17 | 0.22 | 0.0 | 0.0 | 10.83 | 1826 | 236.9 | 321.3 | 23.09 |
| CCL20 | 0.17 | 2.0 | 1.5 | < LLOD | 77.23 | 5.776 | 9.11 | 0.9189 |
| CCL22 | 1.22 | 0.0 | 0.0 | 18.48 | 4227 | 1140 | 1273 | 57.17 |
| CCL26 | 1.77 | 50.0 | 37.6 | < LLOD | 688.7 | 3.967 | 14.41 | 5.337 |
| CXCL10 | 0.37 | 0.0 | 0.0 | 4.872 | 1260 | 272.1 | 334.4 | 18.22 |
| IL-1α | 0.09 | 93.0 | 69.9 | < LLOD | 115.6 | 0.09 | 1.257 | 0.881 |
| IL-1β | 0.05 | 112.0 | 84.2 | < LLOD | 15.31 | 0.05 | 0.1949 | 0.1158 |
| IL-2 | 0.09 | 83.0 | 62.4 | < LLOD | 1.605 | 0.09 | 0.1494 | 0.01548 |
| IL-4 | 0.02 | 64.0 | 48.1 | < LLOD | 0.2262 | 0.02105 | 0.03813 | 0.002428 |
| IL-5 | 0.14 | 97.0 | 72.9 | < LLOD | 9.834 | 0.14 | 0.271 | 0.07345 |
| IL-6 | 0.06 | 5.0 | 3.8 | < LLOD | 55.21 | 0.5437 | 1.519 | 0.4598 |
| IL-7 | 0.12 | 12.0 | 9.0 | < LLOD | 70.59 | 5.794 | 7.456 | 0.7801 |
| IL-8 | 0.07 | 0.0 | 0.0 | 1.814 | 5871 | 16.34 | 162.6 | 46.4 |
| IL-10 | 0.04 | 0.0 | 0.0 | 0.07298 | 2.354 | 0.2392 | 0.3761 | 0.03252 |
| IL-12p40 | 0.33 | 0.0 | 0.0 | 2.045 | 228 | 71.79 | 85.12 | 3.865 |
| IL-12p70 | 0.11 | 93.0 | 69.9 | < LLOD | 0.9872 | 0.11 | 0.148 | 0.009189 |
| IL-13 | 0.24 | 53.0 | 39.8 | < LLOD | 17.68 | 0.5758 | 1.218 | 0.1816 |
| IL-15 | 0.15 | 0.0 | 0.0 | 0.5325 | 2.339 | 1.28 | 1.312 | 0.02849 |
| IL-16 | 2.83 | 0.0 | 0.0 | 33.5 | 566.7 | 146 | 158 | 7.118 |
| IL-17A | 0.10 | 0.0 | 0.0 | 0.1009 | 23.82 | 0.521 | 0.919 | 0.1822 |
| IL_21 | 0.22 | 109.0 | 82.0 | < LLOD | 13.97 | 0.22 | 0.7458 | 0.1763 |
| IL_22 | 0.39 | 23.0 | 17.3 | < LLOD | 99.79 | 0.6546 | 2.713 | 1.034 |
| IL_23 | 0.94 | 126.0 | 94.7 | < LLOD | 11.92 | 0.94 | 1.058 | 0.08841 |
| IL_27 | 7.03 | 0.0 | 0.0 | 254.5 | 4200 | 932.4 | 1046 | 52.37 |
| IL_31 | 0.07 | 123.0 | 92.5 | < LLOD | 0.9644 | 0.07 | 0.07921 | 0.007033 |
| IFN-γ | 0.37 | 0.0 | 0.0 | 0.563 | 42.35 | 2.959 | 4.77 | 0.5279 |
| TNF-α | 0.34 | 0.0 | 0.0 | 0.3455 | 11.68 | 1.454 | 1.697 | 0.1071 |
| TNF-β | 0.08 | 24.0 | 18.0 | < LLOD | 1.014 | 0.1687 | 0.2043 | 0.01274 |
| GM-CSF | 0.16 | 85.0 | 63.9 | < LLOD | 0.5646 | 0.16 | 0.2074 | 0.007937 |
| VEGF-A | 1.12 | 0.0 | 0.0 | 3.171 | 1769 | 103.2 | 150.2 | 16.37 |
| sICAM-1 | 1.94 | 0.0 | 0.0 | 260732 | 1145000 | 472652 | 512210 | 13857 |
| sVCAM-1 | 6.00 | 0.0 | 0.0 | 307333 | 1569000 | 632323 | 653064 | 15297 |
| CRP | 1.33 | 0.0 | 0.0 | 51602 | 103500000 | 1660000 | 6861000 | 1256000 |
| SAA | 10.90 | 0.0 | 0.0 | 17181 | 280300000 | 2005000 | 13220000 | 3720000 |
| S100b | 1.1 | 122 | 91.7 | < LLOD | 875.4 | 0 | 10.54 | 7.339 |
| GFAP | 1.1 | 124 | 93.2 | < LLOD | 134.3 | 0 | 2.929 | 1.315 |

Fig. 2

| Biomarker | BD (mean ± SD) | MDD (mean ± SD) | Effect size | p-value | FDR |
|---|---|---|---|---|---|
| CCL2 | 283.7 ± 20.4 | 284.9 ± 14.6 | -0.07 | 0.94 | 0.94 |
| CCL3 | 25.2 ± 7.6 | 16.45 ± 1.1 | 2.02 | 0.90 | 0.94 |
| CCL4 | 105.0 ± 11.9 | 101.2 ± 6.5 | 0.41 | 0.80 | 0.89 |
| CCL11 | 173.3 ± 18.4 | 201.9 ± 18.1 | -1.57 | 0.63 | 0.86 |
| CCL13 | 143.6 ± 14.6 | 139 ± 7.4 | 0.42 | 0.77 | 0.89 |
| CCL17 | 300.5 ± 46.9 | 330.3 ± 26.3 | -0.81 | 0.32 | 0.54 |
| CCL20 | 9.8 ± 1.6 | 8.7 ± 1.1 | 0.87 | 0.15 | 0.39 |
| CCL22 | 1329.0 ± 106.6 | 1249 ± 67.9 | 0.92 | 0.66 | 0.86 |
| CXCL10 | 337.5 ± 32.3 | 333.1 ± 22.2 | 0.16 | 0.82 | 0.89 |
| IL-6 | 2.5 ± 1.4 | 1.11 ± 0.3 | 1.63 | 0.03 | 0.19 |
| IL-7 | 7.4 ± 1.1 | 7.5 ± 1.0 | -0.03 | 0.48 | 0.70 |
| IL-8 | 285.6 ± 147.3 | 109.7 ± 19.0 | 2.12 | 0.04 | 0.21 |
| IL-10 | 0.5 ± 0.1 | 0.31 ± 0.03 | 3.55 | 0.01 | 0.09 |
| IL-12p40 | 91.8 ± 6.4 | 10000 ± 4.8 | 1.71 | 0.15 | 0.39 |
| IL-15 | 1.36 ± 0.04 | 1.29 ± 0.04 | 1.86 | 0.18 | 0.39 |
| IL-16 | 161.1 ± 11.3 | 156.6 ± 9.0 | 0.44 | 0.42 | 0.66 |
| IL-17A | 1.6 ± 0.6 | 0.6 ± 0.1 | 3.09 | 0.0004 | 0.01 |
| IL-27 | 1189.0 ± 110.2 | 979.7 ± 54.2 | 2.55 | 0.08 | 0.28 |
| IFN-γ | 5.6 ± 1.1 | 4.4 ± 0.6 | 1.32 | 0.07 | 0.28 |
| TNF-α | 2.1 ± 0.3 | 1.5 ± 0.1 | 3.12 | 0.02 | 0.17 |
| VEGF-A | 144.7 ± 16.9 | 152.6 ± 22.3 | -0.40 | 0.29 | 0.51 |
| sICAM-1 | 548592 ± 27846 | 496136 ± 15423 | 2.42 | 0.11 | 0.34 |
| sVCAM-1 | 654942 ± 27772 | 652033 ± 18229 | 0.13 | 0.75 | 0.89 |
| CRP | 9435000 ± 2440000 | 5698000 ± 1436000 | 1.93 | 0.27 | 0.51 |
| SAA | 22640000 ± 9988000 | 9051000 ± 3032000 | 2.09 | 0.19 | 0.39 |

Fig. 3

| | Model 1 | | Model 2 | |
|---|---|---|---|---|
| | Proportion of runs | Weighted Coefficients ± SD | Proportion of runs | Weighted Coefficients ± SD |
| Intercept | 1 | -0.818 ± 0.012 | 1 | -0.814 ± 0.017 |
| Sex (male) | 0.43 | -0.076 ± 0.004 | | |
| Age | 0.16 | -0.008 ± 0.001 | | |
| BMI | 0.41 | 0.037 ± 0.001 | | |
| HDRS-17 | 0.48 | 0.066 ± 0.003 | | |
| Smoking | 0.37 | -0.087 ± 0.006 | | |
| Benzodiazepines | 0.99 | 0.326 ± 0.017 | 1 | 0.613 ± 0.012 |
| Antidepressants | 0.97 | -0.284 ± 0.016 | 1 | -0.492 ± 0.011 |
| Neuroleptics or atypical antipsychotics | 0.98 | 0.175 ± 0.006 | 1 | 0.291 ± 0.01 |
| Other antipsychotic treatments | 0.98 | 0.235 ± 0.009 | 0.9995 | 0.338 ± 0.009 |
| Lithium | 0.89 | 0.177 ± 0.009 | 0.9785 | 0.257 ± 0.016 |
| CCL2 | 0.19 | -0.034 ± 0.001 | | |
| CCL3 | 0.52 | 0.042 ± 0.001 | | |
| CCL4 | 0.13 | -0.023 ± 0.001 | | |
| CCL11 | 0.52 | -0.062 ± 0.002 | | |
| CCL13 | 0.23 | 0.032 ± 0.002 | | |
| CCL17 | 0.25 | -0.056 ± 0.002 | | |
| CCL20 | 0.21 | 0.005 ± 0.004 | | |
| CCL22 | 0.30 | 0.056 ± 0.003 | | |
| CXCL10 | 0.22 | -0.042 ± 0.004 | | |
| IL-6 | 0.21 | 0.009 ± 0.001 | | |
| IL-7 | 0.17 | 0.046 ± 0.002 | | |
| IL-8 | 0.53 | 0.03 ± 0.00 | | |
| IL-10 | 0.84 | 0.096 ± 0.003 | 0.97 | 0.19 ± 0.005 |
| IFN-$\gamma$ | 0.25 | 0.006 ± 0.004 | | |
| IL-12p40 | 0.25 | 0.016 ± 0.002 | | |
| IL-15 | 0.62 | 0.061 ± 0.002 | | |
| IL-16 | 0.14 | -0.046 ± 0.003 | | |
| IL-17 | 1.00 | 0.161 ± 0.005 | 1 | 0.312 ± 0.002 |
| IL-27 | 0.64 | 0.056 ± 0.002 | | |
| TNF-$\alpha$ | 0.85 | 0.091 ± 0.003 | 0.911 | 0.124 ± 0.005 |
| VEGF-A | 0.09 | -0.02 ± 0.001 | | |
| sICAM-1 | 0.78 | 0.092 ± 0.004 | | |
| sVCAM-1 | 0.09 | 0.021 ± 0.002 | | |
| CRP | 0.33 | 0.067 ± 0.004 | | |
| SAA | 0.67 | 0.058 ± 0.002 | | |

Fig. 4

Europäisches
Patentamt

European
Patent Office

Office européen
des brevets

## EUROPEAN SEARCH REPORT

Application Number

EP 18 19 8714

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YUYAN CHENG ET AL: "A pre-conditioning stress accelerates increases in mouse plasma inflammatory cytokines induced by stress", BMC NEUROSCIENCE, BIOMED CENTRAL, LONDON, GB, vol. 16, no. 1, 7 May 2015 (2015-05-07), page 31, XP021220840, ISSN: 1471-2202, DOI: 10.1186/S12868-015-0169-Z * abstract; figure 4 * | 1,2,4-14 | INV. G01N33/68 |
| X | M Kunz: "Serum levels of IL-6, IL-10 and TNF-[alpha] in patients with bipolar disorder and schizophrenia: differences in pro- and anti-inflammatory balance.", , 1 September 2011 (2011-09-01), pages 268-274, XP055534387, Retrieved from the Internet: URL:http://www.ncbi.nlm.nih.gov/pubmed/21971780 [retrieved on 2018-12-14] p 269, col 2, last lines; * abstract * | 1,5,6, 8-13 | |
| X | WEI ZOU ET AL: "Changes in the serum levels of inflammatory cytokines in antidepressant drug-naïve patients with major depression", PLOS ONE, vol. 13, no. 6, 1 June 2018 (2018-06-01), page e0197267, XP055534864, DOI: 10.1371/journal.pone.0197267 p 3, para 1; * abstract * | 1,2,5,6, 9-13 | |

TECHNICAL FIELDS
SEARCHED     (IPC)

G01N

-/--

The present search report has been drawn up for all claims

| Place of search | Date of completion of the search | Examiner |
|---|---|---|
| Munich | 17 December 2018 | Bigot-Maucher, Cora |

EPO FORM 1503 03.82 (P04C01)

Europäisches
Patentamt

European
Patent Office

Office européen
des brevets

# EUROPEAN SEARCH REPORT

Application Number

EP 18 19 8714

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NORA HAMDANI ET AL: "Relationship between Toxoplasma gondii infection and bipolar disorder in a French sample", JOURNAL OF AFFECTIVE DISORDERS., vol. 148, no. 2-3, 1 June 2013 (2013-06-01), pages 444-448, XP055534856, NL ISSN: 0165-0327, DOI: 10.1016/j.jad.2012.11.034 * p 447, col 1, para 2 * | 6 | |
| A | UPTHEGROVE RACHEL ET AL: "Cytokine function in medication-naive first episode psychosis: A systematic review and meta-analysis", SCHIZOPHRENIA RESEARCH, vol. 155, no. 1, 2014, pages 101-108, XP028648807, ISSN: 0920-9964, DOI: 10.1016/J.SCHRES.2014.03.005 * p 104, col 1, para 1 * | 6 | |
| A | R.M. HIRSCHFELD ET AL: "Differential diagnosis of bipolar disorder and major depressive disorder", JOURNAL OF AFFECTIVE DISORDERS., vol. 169, 1 December 2014 (2014-12-01), pages S12-S16, XP055534859, NL ISSN: 0165-0327, DOI: 10.1016/S0165-0327(14)70004-7 * the whole document * | 1-14 | |

TECHNICAL FIELDS
SEARCHED (IPC)

-/--

The present search report has been drawn up for all claims

| Place of search | Date of completion of the search | Examiner |
|---|---|---|
| Munich | 17 December 2018 | Bigot-Maucher, Cora |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 18 19 8714

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ASHLEY L. COMES ET AL: "Proteomics for blood biomarker exploration of severe mental illness: pitfalls of the past and potential for the future", TRANSLATIONAL PSYCHIATRY, vol. 8, no. 1, 16 August 2018 (2018-08-16), XP055534745, DOI: 10.1038/s41398-018-0219-2 * the whole document * | 1-14 | |
| T | BARBOSA IZABELA GUIMARÃES ET AL: "Predictors of cognitive performance in bipolar disorder: The role of educational degree and inflammatory markers.", JOURNAL OF PSYCHIATRIC RESEARCH NOV 2018, vol. 106, November 2018 (2018-11), pages 31-37, XP002787448, ISSN: 1879-1379 * abstract * | | |
| T | HIROHITO TSUBOI ET AL: "Elevated Levels of Serum IL-17A in Community-Dwelling Women with Higher Depressive Symptoms", BEHAVIORAL SCIENCES, vol. 8, no. 11, 4 November 2018 (2018-11-04), page 102, XP055534398, DOI: 10.3390/bs8110102 * the whole document * | | TECHNICAL FIELDS SEARCHED (IPC) |

The present search report has been drawn up for all claims

| Place of search | Date of completion of the search | Examiner |
|---|---|---|
| Munich | 17 December 2018 | Bigot-Maucher, Cora |

EPO FORM 1503 03.82 (P04C01)